# EUROPÄISCHE PATENTSCHRIFT

(11) **EP 0 809 566 B1**
(45) Veröffentlichungstag und Bekanntmachung des Hinweises auf die Patenterteilung: **25.04.2001**
(21) Anmeldenummer: 96943016.4
(22) Anmeldetag: 15.12.1996
(51) Int. Cl.: B29C 41/14, A61F 6/04, B29C 33/42, B29C 41/22

(54) **HERSTELLUNG EINES PROPHYLAKTIKUMS**
PRODUCTION OF A PROPHYLACTIC
FABRICATION D'UN PRESERVATIF

(30) Priorität: 15.12.1995 DE 19546985
(43) Veröffentlichungstag der Anmeldung: 03.12.1997
(73) Patentinhaber: Innovation & Art Gmbh, 81543 München (DE)
(72) Erfinder: SCHOLL, Thomas, D-81543 München (DE)
(74) Vertreter: Söffge, Karen
(86) Internationale Anmeldenummer: DE9602405
(87) Internationale Veröffentlichungsnummer: WO9722454

(56) Entgegenhaltungen:
- EP-A- 0 557 625
- WO-A-94/13231
- WO-A-95/25622
- AT-B- 123 532
- DE-A- 1 766 519
- DE-A- 2 459 473
- PATENT ABSTRACTS OF JAPAN vol. 010, no. 333 (M-534), 12.November 1986 & JP 61 136762 A (TOSHIBA CORP), 24.Juni 1986,
- PATENT ABSTRACTS OF JAPAN vol. 013, no. 025 (M-787), 20.Januar 1989 & JP 63 237863 A (KYOCERA CORP), 4.Oktober 1988,

## Beschreibung

Die vorliegende Erfindung befaßt sich mit der Herstellung eines geformten Prophylaktikums mit besonderen Rundungen, bei dem einerseits die spezielle Form des Tauchwerkzeugs und andererseits das Herstellungsverfahren eine bedeutende Rolle spielen.

Ein derartiges Tauchwerkzeug und Verfahren zur Herstellung eines Prophylaktikums wurde bereits in der internationalen Anmeldung WO 95/25622 bzw. der DE 4409449 C1 vorgestellt und ausführlich beschrieben. Mit diesem bekannten Tauchwerkzeug wird ein Prophylaktikum hergestellt, das aus einem länglichen, zylindrischen Schaft mit einem durch einen s-förmigen Abschnitt und ein daran anschließendes Reservoirteil verschlossenen Ende besteht. Es ist eine Vertiefung, die bei Gebrauch im Bereich der Eichel zur Anlage kommt, vorgesehen, die aus zwei mit den kleinen Deckflächen aneinanderstoßenden Kegelstümpfen gebildet ist. Die Vertiefung hat einen Öffnungswinkel im Bereich zwischen 60° und 120°. Das bekannte Prophylaktikum wird hergestellt durch dreimaliges Eintauchen in eine Latexlösung. Zwischen jedem Tauchvorgang wird ein Trocknungsofen durchlaufen und am Ende des letzten Trocknungsvorgangs wird das bekannte Prophylaktikum mittels gegensinnig rotierender Bürsten abgestreift.

Ferner sind ähnliche Verfahren und Vorrichtungen zur Herstellung dünnwandiger Formkörpber, insbesondere aus Naturkautschukverbindungen im Stand der Technik wohlbekannt.

Bei den bekannten Herstellungsmethoden eines Prophylaktikums wird das Tauchwerkzeug bei schwierigen Formgebungen drei Tauchvorgängen unterzogen. Die drei Tauchvorgänge sind besonders wichtig bei Formgebungen, die an bestimmten Stellen des Tauchwerkzeugs relativ kleine Krümmungsradien aufweisen, so daß an diesen kleinen Krümmungsradien das aufzubringende elastische Material (Latex) ungleichmäßig verteilt wird, worin häufig die Ursache für Reiß- und Bruchstellen zu suchen ist und das Prophylaktikum nicht den Anforderungen der Prüfbehörden entspricht.

Da es allgemein schwierig ist, ein Prophylaktikum herzustellen, das den hohen Anwendungsanforderungen bezüglich des guten Sitzes und der leichten Handhabung gerecht wird, wurden im Stand der Technik verschiedene Formen, insbesondere im oberen Teil des Prophylaktikums vorgeschlagen, die die anstehenden Probleme lösen sollen. Hierzu zählt auch die im Stand der Technik aus dem Jahre 1967 bekannte US-Druckschrift PS 1,142,443, aus der ein Prophylaktikum bekannt ist, das einen zylindrischen Teil und einen oberen geformten Teil aufweist. Der obere geformte Teil dieses bekannten Prophylaktikums weist eine Verengung auf, die so ausgebildet ist, daß sie einen sicheren Sitz auf dem erigierten männlichen Organ gewährleisten soll. Dieses Prophylaktikum besitzt jedoch keinerlei Reservoirteil am oberen Ende des Prophylaktikums, wie dies heutzutage bei jedem handelsüblichen Prophylaktikum vorzufinden ist. Bereits aus diesem Grunde sind derartige Prophylaktika nicht mehr zulässig.

Ein weiterer entscheidender Nachteil besteht darin, daß der Krümmungsradius der Einengung des Prophylaktikums im oberen Teil gleichmäßig durch die gesamte Vertiefung verläuft und somit keinerlei Gewähr dafür bietet, daß eine hinreichend gute Anpassung an die anatomischen Verhältnisse des männlichen Penis gewährleistet ist.

Ferner ist aus der österreichischen Patentschrift Nr. 123 532 ein Präservativ aus dem Jahre 1929 aus Gummi bekannt geworden, der im oberen geformten Teil eine Einschnürung (a) aufweist und aus zwei mit ihren kleinen Deckflächen aneinanderstoßenden Kegelstümpfen besteht, wobei die Stoßstellen der Kegelstümpfe glatt sind. Die in dieser Patentschrift gezeigten Formen der Tauchwerkzeuge weisen jedoch stark ausgeprägte S-Kurven der Mantelflächen der Kegelstümpfe auf, so daß eine Realisierung für den praktischen Gebrauch eines derartigen Prophylaktikums problematisch ist.

Aus der CH 96564 ist ein Kurzkondom mit einem keulenartigem Reservoirteil bekannt, das auf den Penis eines Benutzers aufgeklebt werden muß. In der CH 96564 werden keine Maßnahmen offenbart, aus denen hervorgeht, daß das keulenartige Reservoirteil durch Druck- und Zugspannungen, die durch eine besondere Formgebung hervorgerufen wird, sich in eine aufrechte Gebrauchslage aufrichtet.

Ziel der vorliegenden Erfindung ist es, ein neuartiges Prophylaktikum mit ausgeprägten Formen im oberen Teil zu schützen, wobei die Krümmungsradien der einzelnen Rundungen an den vorbestimmten Stellen eine entscheidende Rolle spielen, die die inneren Druck- und Zugspannungen des Prophylaktikums sowohl im aufgerollten als auch im abgerollten Zustand entscheidend beeinflussen.

Daher ist es Aufgabe der vorliegenden Erfindung, ein Prophylaktikum bereitzustellen, das einen optimalen Sitz und eine sicher Handhabung gewährleistet.

Diese Aufgabe wird erfindungsgemäß durch die in den Ansprüchen 1, 2 und 11 genannten Merkmale gelöst.

Das erfindungsgemäße Tauchwerkzeug zur Herstellung eines Prophylaktikums ist dadurch gekennzeichnet, daß
- der Durchmesser (D5) des Tauchwerkzeugs (1) an der engsten Stelle der Vertiefung (6) zwischen 15 und 25 mm beträgt;
- der Krümmungsradius (R1) an der engsten Stelle (D5) der Vertiefung (6) zwischen 17 und 20 mm beträgt;
- der Öffnungswinkel des oberen, zum Reservoirteil (4) hin geöffneten Kegelstumpfes (9) 50° beträgt;
- der Öffnungswinkel des unteren Kegelstumpfes (8) 40° beträgt;
- der Krümmungsradius an der Übergangsstelle vom zylindrischen (2) zur Vertiefung (6) im geformten Abschnitt (3) konvex und größer 25 mm ist;
- die Gesamtlänge von der Vertiefung (6) bis zum Eingang in das Reservoirteil (4) zwischen 45 mm und 65 mm beträgt;
- der größte Durchmesser (D2) des geformten Abschnitts (3) im Bereich zwischen Vertiefung (6) und Eingangsbereich in das Reservoirteil (4) zwischen 33 und 35 mm beträgt.

Dabei ist der kleinste Krümmungsradius, d.h. die stärkste Krümmung der Vertiefung etwa in der Mitte der Vertiefung vorzufinden. Im Anschluß an diese Krümmung verlaufen nach beiden Seiten schwach ausgebildete S-Kurven, die die Mantelflächen zweier mit ihren kleinen Deckflächen aneinanderstoßenden Kegelstümpfe darstellen.

Eine weitere erfindungsgemäße Ausführungsform ist dadurch gekennzeichnet, daß die Vertiefung in der Nähe des engsten Durchmessers (D5) mindestens eine Ringnut aufweist, so daß hierdurch am fertigen Prophylaktikum mindestens ein Ring entsteht, dessen Masse sehr viel größer ist als die Masse des elastischen Materials in seiner Umgebung. Vorteilhaft für das erfindungsgemäße Prophylaktikum sind zwei derartige Ringnuten, die ca. 1,5 mm beabstandet sind.

Besonders vorteilhaft für das erfindungsgemäße Prophylaktikum ist das am oberen Ende angeordnete sogenannte Reservoirteil, das erfindungsgemäß keulenartig ausgebildet ist. Infolge der speziellen Rundungen und Maßverhältnisse des erfindungsgemäßen Reservoirteils hat dieses Reservoirteil die Eigenschaft, daß es nach dem Herausziehen aus der vertriebsmäßigen Verpackung aufrecht in Gebrauchsrichtung steht. Dieses Aufrichten des Reservoirteils im aufgerollten Zustand des Prophylaktikums ist nur möglich, wenn innerhalb des Prophylaktikums durch dessen Formgebung im geformten Abschnitt des Prophylaktikums Zug und Druckspannungen entstehen, die so gerichtet sind, daß das Reservoirteil in Gebrauchsrichtung mit seiner Mittelachse senkrecht zur Ebene des aufgerollten Prophylaktikums steht.

Darüber hinaus hat die spezielle Formgebung mit ihren glatten Rundungen den Vorteil, daß sich das elastische Material beim Tauchvorgang in den einzelnen Tauchbecken gleichmäßig über das Tauchwerkzeug verteilen, und somit keinerlei Dünnstellen, die häufig die Ursache von Mangelhaftigkeit des Prophylaktikums sind, darstellt.

Vorteilhaft und erfindungsgemäß ist es auch, daß der Übergang vom größten Durchmesser des Tauchwerkzeugs im geformten Abschnitt zum Reservoirteil hin nicht steil und gerade verläuft, sondern eine nach unten geneigte geschwungene S-Kurve darstellt.

Aufgrund der erfindungsgemäßen Formgebung des Tauchwerkzeugs ist es möglich, ein erfindungsgemäßes Verfahren zur Herstellung des Prophylaktikums bereitzustellen, bei dem das Tauchwerkzeug in zwei Tauchvorgängen rotierend um seine Längsachse in einem bestimmten Winkel zur Oberfläche in das aufzubringende elastische Material (Latex) eingetaucht wird. Nach den Tauchvorgängen ist jeweils ein Trocknungsvorgang in einem speziellen Trocknungsofen vorgesehen.

Ein weiterer erfindungsgemäßer Aspekt im erfindungsgemäßen Verfahren zur Herstellung des Prophylaktikums besteht darin, daß das Tauchwerkzeug auf der Oberfläche Ringnuten mit Hilfe mindestens einer scheibenförmigen feinkörnigen Diamant-Schleifspitze eingeschliffen werden, deren Winkel (α) zwischen 60° und 120° liegt, wobei das vordere Teil der Diamant-Schleifspitze eine Abstumpfung aufweist. Diese Abstumpfung ist besonders notwendig, damit die zu erstellende Ringnut auf der Oberfläche des Tauchwerkzeugs keine scharfen Kanten aufweist, in die sich das elastische Material beim Tauchvorgang festsetzen kann, wodurch besondere Reinigungsvorgänge notwendig würden.

Eine Ausführungsform der Ringnuten ist so zu gestalten, daß sie symmetrisch zur Mittelachse der Ringnut verläuft.

Eine andere Variante der erfindungsgemäß geschliffenen Ringnuten besteht darin, daß die Ringnuten asymmetrisch zur Mittelachse derart geformt werden, daß die Wand der Ringnut links der Achse (in Fig. 11) steiler als die Wand rechts der Achse verläuft. Ein wichtiger Aspekt in der Asymmetrie der Ringnut ist darin zu sehen, daß das Verhältnis der Abstände (A, A') zur Mittelachse der Ringnut etwa 1:2 beträgt. Hierdurch ist ein günstiger Verlauf des aufzubringenden Latex oder eines anderen elastischen Materials gewährleistet.

Im allgemeinen stellt das Abstreifen des fertigen Prophylaktikums vom Tauchwerkzeug bei ausgeprägten Formgebungen ein Problem dar. Dieses Problem wird erfindungsgemäß dadurch gelöst, daß beim Abstreifvorgang durch spezielle gegensinnig rotierende Bürsten mindestens ein Wasserstrahl so ausgerichtet ist, daß er hinter dem von den Bürsten gebildeten Rollring während des Abstreifvorgangs auf das Tauchwerkzeug einwirkt. Hierdurch wird zwischen dem fertigen Prophylaktikum und dem Tauchwerkzeug ein hauchdünner Wasserfilm erzeugt, der das Abstreifen des fertigen Prophylaktikums überhaupt ermöglicht.

Weitere erfindungsgemäße Merkmale sind den Unteransprüchen zu entnehmen.

Im nun folgenden wird anhand von Zeichnungen die vorliegende Erfindung im Detail näher erläutert. Es zeigt:
- Fig. 1: eine schematische Darstellung eines erfindungsgemäßen Tauchwerkzeugs (1) in einer Seitenansicht, die die prinzipielle Formgebung des oberen Teils (3) des Tauchwerkzeugs (1) wiedergibt;
- Fig. 2: eine schematische Darstellung des Tauchwerkzeugs (1) in Seitenansicht mit verschiedenen Maßebenen (10), die für die Bemaßung des Tauchwerkzeugs (1) wichtig sind;
- Fig. 3: eine Prinzipdarstellung des Reservoirteils (4) des Tauchwerkzeugs (1) mit seiner speziellen Formgebung des Übergangs (15) vom großen Durchmesser (D2) zum Reservoirteil (4);
- Fig. 4: eine Prinzipdarstellung des aufgerollten Prophylaktikums mit dem aufrecht in Gebrauchsrichtung stehenden Reservoirteil (4);
- Fig. 5: eine Prinzipdarstellung der erfindungsgemäßen Tauch- und Trocknungsvorrichtung zur Herstellung eines Prophylaktikums;
- Fig. 6: eine schematische Darstellung der Abstreifvorrichtung mit den erfindungsgemäßen Wasserstrahlen (27, 28), die hinter dem abgebürsteten Prophylaktikum auf das Tauchwerkzeug (1) einwirken.
- Fig. 7: einen Querschnitt durch die Schleifscheibe (30) mit den erfindungsgemäßen Diamant-Schleifspitzen (31, 32);
- Fig. 8: eine Vergrößerung der erfindungsgemäßen Diamant-Schleifspitzen (31, 32);
- Fig. 9: eine Vergrößerung einer Diamant-Schleifspitze (31) mit fünf gleichen erfindungsgemäßen Schleifspitzen (32);
- Fig. 10a: eine symbolisierte Anordnung von symmetrischen Ringnuten (17, 18) auf der Oberfläche des Tauchwerkzeugs (1);
- Fig. 10b: eine vergrößerte symmetrische Ringnut (17, 18);
- Fig. 11a: eine schematische Darstellung einer asymmetrischen Ringnut (34) im Graben (6) eines geformten Tauchwerkzeugs (1);
- Fig. 11b: eine vergrößerte Ringnut (34).

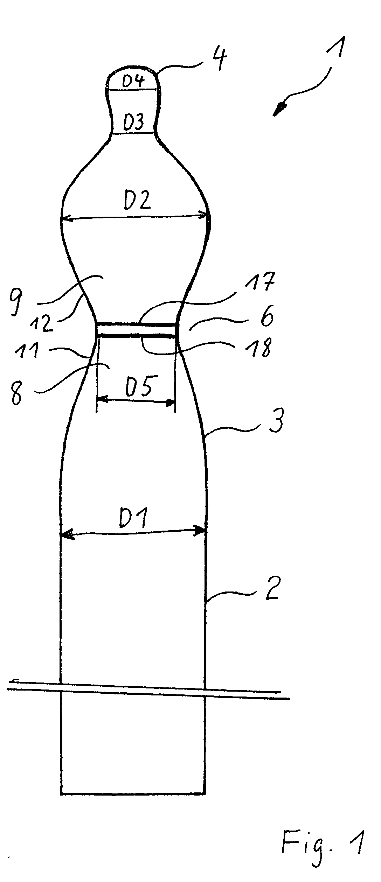

In Fig. 1 ist eine schematische Darstellung des Tauchwerkzeugs 1 in einer Seitenansicht dargestellt. Im vorliegenden Ausführungsbeispiel besteht das Tauchwerkzeug aus Glas. Aber ebensogut kann das Tauchwerkzeug aus einem anderen Werkstoff wie beispielsweise Porzellan, Kunststoff, Keramik oder dergleichen gefertigt werden. Das Tauchwerkzeug 1 setzt sich schematisch aus einem langgestreckten zylindrischen Teil 2 und einem oberen geformten Teil 3 zusammen. Der Durchmesser D1 des zylindrischen Teils 2 beträgt ca. 34 mm. An den langgestreckten unteren zylindrischen Teil 2 schließt der obere geformte Teil 3 ohne erkennbaren Übergang nahtlos an. Der Übergang vom zylindrischen zum geformten Teil erfolgt mit einem verhältnismäßig großen Krümmungsradius. Der obere geformte Teil 3 des Tauchwerkzeugs 1 setzt sich wiederum aus drei Hauptbestandteilen zusammen. Diese sind zwei kegelstumpfartige Gebilde 8 und 9, deren Mantelflächen eine geschwungene S-Kurve beschreiben. Die beiden kegelstumpfartigen Gebilde 8 und 9 stoßen mit ihren kleinen Deckflächen nahtlos aneinander. An der Stoßstelle hat die erfindungsgemäße Vertiefung 6 ihren tiefsten Punkt und somit auch den engsten Durchmesser D5. Der Durchmesser D2 des Kegelstumpfs 9 ist nahezu der gleiche wie der Durchmesser D1 des langgestreckten zylindrischen Teils 2 des Tauchwerkzeugs 1. An den Stoßstellen der Kegelstümpfe 8, 9 ist der Krümmungsradius der Vertiefung 6 am kleinsten und hat somit hier die stärkste Krümmung, verglichen zu den Krümmungen der Mantelflächen 11, 12 der Kegelstümpfe 8, 9. Vom Durchmesser D2 des Kegelstumpfs 9 beginnt praktisch der Übergang zum sogenannten Reservoirteil 4. Wegen der Wichtigkeit des Überganges wird dieser weiter unten näher beschrieben. Der Durchmesser D3 des Eingangs des Reservoirteils ist geringfügig kleiner als der Durchmesser D4 im oberen Bereich des Reservoirteils 4.

In einer Ausführungsform des erfindungsgemäßen Tauchwerkzeugs 1 kann dieses in der Nähe der engsten Stelle der Vertiefung 6 mindestens eine Ringnut bzw. einen Ring aufweisen. Im vorliegenden Falle befinden sich zwei Ringnuten 17, 18 in der Vertiefung 6. Die Ringnuten 17, 18 sind etwa 1,5 mm voneinander beabstandet.

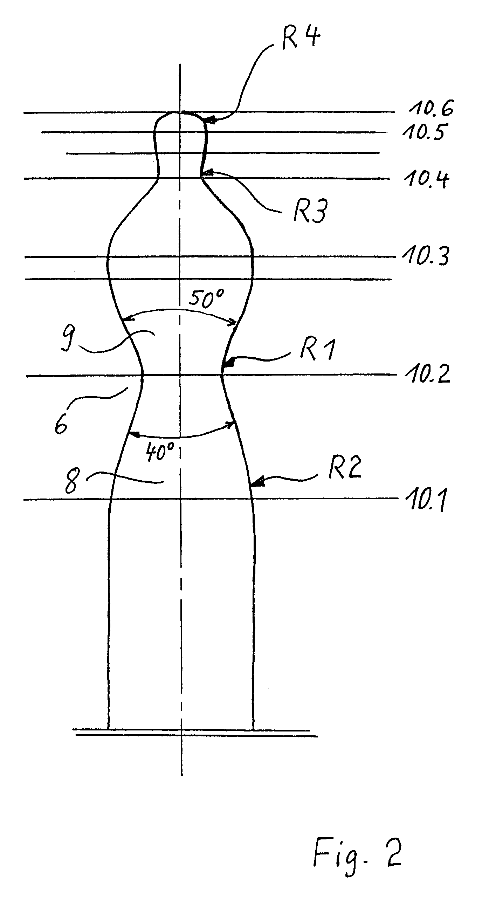

In Fig. 2 ist ein weiteres erfindungsgemäßes Tauchwerkzeug 1 in Seitenansicht schematisch mit verschiedenen Maßebenen 10.1 bis 10.6 dargestellt. Die Maßebenen 10 sind gedachte Linien, die für die Bemaßung des Tauchwerkzeugs 1 dieses Ausführungsbeispiels notwendig sind. Die Maßebene 10.2 verläuft deckungsgleich parallel zum Durchmesser D5 der engsten Stelle der Vertiefung 6. Ein Idealmaß des Durchmessers D5 = 20 mm, kann jedoch zwischen 15 und 25 mm variieren. Die Maßebene 10.3 verläuft durch den Durchmesser D2, der etwa 34 mm beträgt. Die Maßebene 10.4 verläuft durch den Durchmesser D3, der etwa 10 mm beträgt, und die Maßlinie 10.5 verläuft durch den Durchmesser D4 = 12 mm. Die Maßlinie 10.6 stellt das Ende des Tauchwerkzeugs 1 dar, was mit dem Ende des Reservoirteils 4 übereinstimmt. Ein vorteilhaftes Maß für die Gesamtlänge des geformten Teils 3 des Tauchwerkzeugs 1 beträgt ca. 100 mm und erstreckt sich von der Maßebene 10.1 zur Maßebene 10.6. Das Maß für den Abstand der Maßebene 10.1 zur Maßebene 10.2 beträgt ca. 33 mm und das Maß für den Abstand der Ebene 10.2 zur Ebene 10.4 beträgt rund 50 mm. Das Maß für den Abstand der Maßebene 10.4 zur Maßebene 10.6 beträgt ca. 16 mm.

Die Öffnungswinkel der Kegelstümpfe sind unterschiedlich, wobei der untere Kegelstumpf 8 einen Öffnungswinkel von 40° und der obere Öffnungswinkel des Kegelstumpfs 9 50° beträgt. Der Krümmungsradius R1 ist im vorliegenden Fall 18 mm, wobei der Krümmungsradius R2 ≥ 25 mm ist. Der Krümmungsradius R3 am Fuße des Reservoirteils 4 in der Nähe des Durchmessers D3 beträgt 12 mm. Der Krümmungsradius R4 am oberen Ende des Reservoirteils 4 ist 6,5 mm. Die Gesamtlänge des Reservoirteils von der Maßebene 10.4 zur Maßlinie 10.6 beträgt 16 mm.

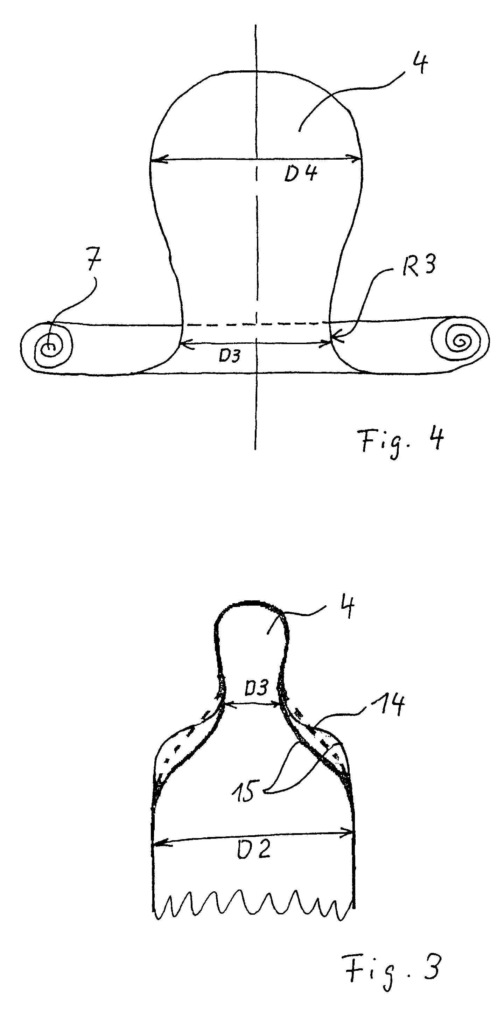

Fig. 3 zeigt den obersten Teil des Tauchwerkzeugs 1, d.h. das Reservoirteil 4 mit den wichtigen Übergängen vom Durchmesser D2 zum unteren Durchmesser D3 des unteren Teils des Reservoirteils 4. Wichtig und erfindungsgemäß ist hier sowohl die besondere Formgebung des keulenartigen Reservoirteils 4 als auch der Übergang 15, der nicht, wie im Stand der Technik, steil verlaufen darf, so wie es mit der gestrichelten Linie 14 angedeutet ist, sondern muß eine geschwungene S-kurvenförmige Linie beschreiben, was letztlich die Spannungen im fertigen aufgerollten Prophylaktikum erzeugt und das Reservoirteil 4 aufrecht zur Ebene des aufgerollten Propylaktikums stehen läßt.

In Fig. 4 ist schematisch ein aufgerolltes Prophylaktikum dargestellt. Infolge der besonderen Formgebung des Übergangs 15 und der Krümmungsradien R3 in der Nähe des Durchmessers D3 des Reservoirteils 4 treten dort infolge der Vorspannung durch den Rollring 7 des aufgerollten Prophylaktikums Spannungen auf, die so gerichtet sind, daß das Reservoirteil 4 im ausgepackten Zustand aufrecht in Gebrauchsrichtung steht.

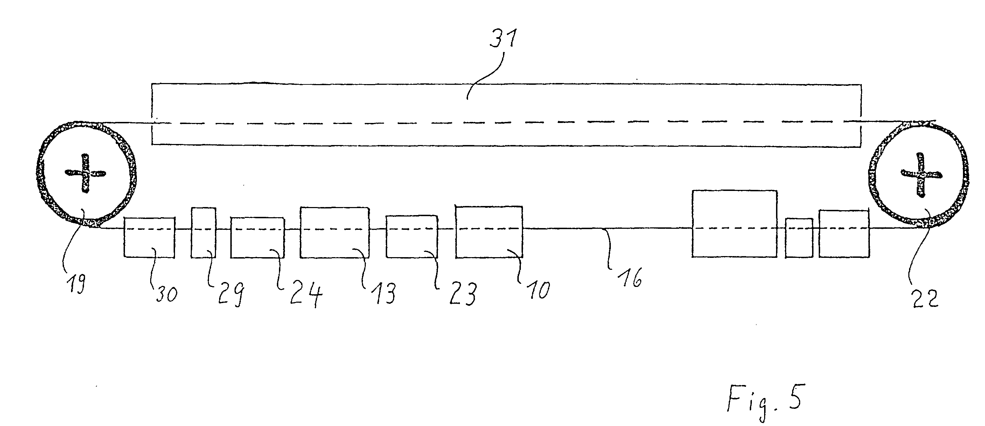

In Fig. 5 ist eine Vorrichtung zur Massenproduktion eines Prophylaktikums schematisch dargestellt. Die Gesamtlänge dieser Anlage von einem Umlenkrad 19 zum anderen 22 beträgt ca. 30 m. Das Tauchwerkzeug 1 ist mit einem nicht näher zu beschreibenden Mechanismus an einem Endlosförderband 16 befestigt, so daß das Tauchwerkzeug 1 mit einer bestimmten Geschwindigkeit die einzelnen Verfahrensschritte durchläuft. Diese sind in der oben erwähnten Druckschrift WO 95/25622 näher beschrieben. Wichtig bei dem erfindungsgemäßen Verfahren ist es, daß hier lediglich zwei Tauchvorgänge in den Tauchbecken 10 und 13 durchgeführt werden. Nachdem das Tauchwerkzeug 1 rotierend durch das Tauchbecken 10 geführt wurde, erfolgt eine Trocknung in der Vorrichtung 23, die von dem Tauchwerkzeug 1 in waagerechter Stellung rotierend durchlaufen wird. Nach dem zweiten Tauchvorgang im Tauchbecken 13 wird mit Hilfe von nicht gezeigten gegensinnig rotierenden Bürsten am offenen Teil des Prophylaktikums ein Rollrand 7 in der Vorrichtung 29 gebildet. Im Anschluß daran wird das fast fertige Prophylaktikum einer Zwischentrocknung in der Vorrichtung 30 unterzogen. Die Vulkanisierstrecke 31 ist ein langgestreckter Ofen, der sich fast über die gesamte Länge der Anlage erstreckt.

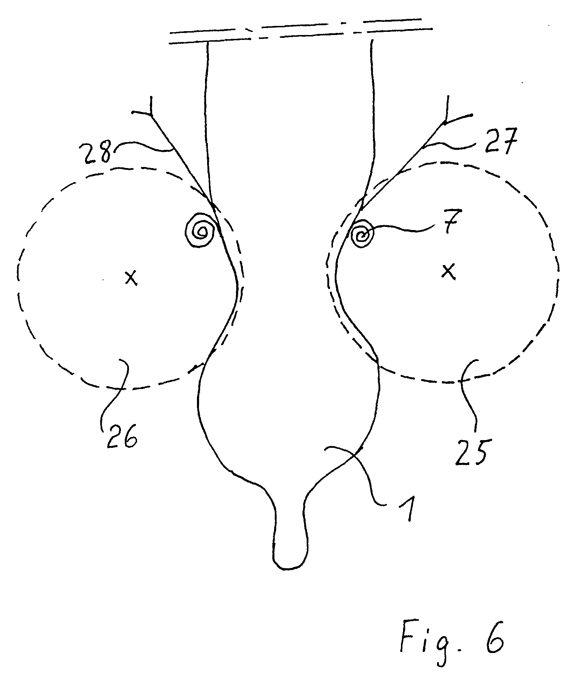

In Fig. 6 ist prinzipiell die Abstreifvorrichtung für das fertige Prophylaktikum vom Tauchwerkzeug 1 dargestellt. Auf das Tauchwerkzeug 1 wirken zwei gegensinnig rotierende Bürsten 25, 26 ein und streifen das Prophylaktikum herunter. Aufgrund des extrem kleinen Durchmessers D5 an der engsten Stelle der Vertiefung 6 ist es schwierig, nur mit Hilfe der Bürsten 25, 26 das fertige Prophylaktikum vom Tauchwerkzeug 1 abzustreifen. Die Lösung dieser Schwierigkeit besteht darin, daß hinter dem gebildeten Rollring 7 mindestens ein Wasserstrahl 27, 28 so auf das Tauchwerkzeug 1 einwirkt, daß sich zwischen dem Prophylaktikum und dem Tauchwerkzeug ein leichter Wasserfilm bildet, der es ermöglicht, das Prophylaktikum abzustreifen. Je nach Bedarf kann der Wasserstrahl 27, 28 gepulst oder kontinuierlich auf das Tauchwerkzeug einwirken.

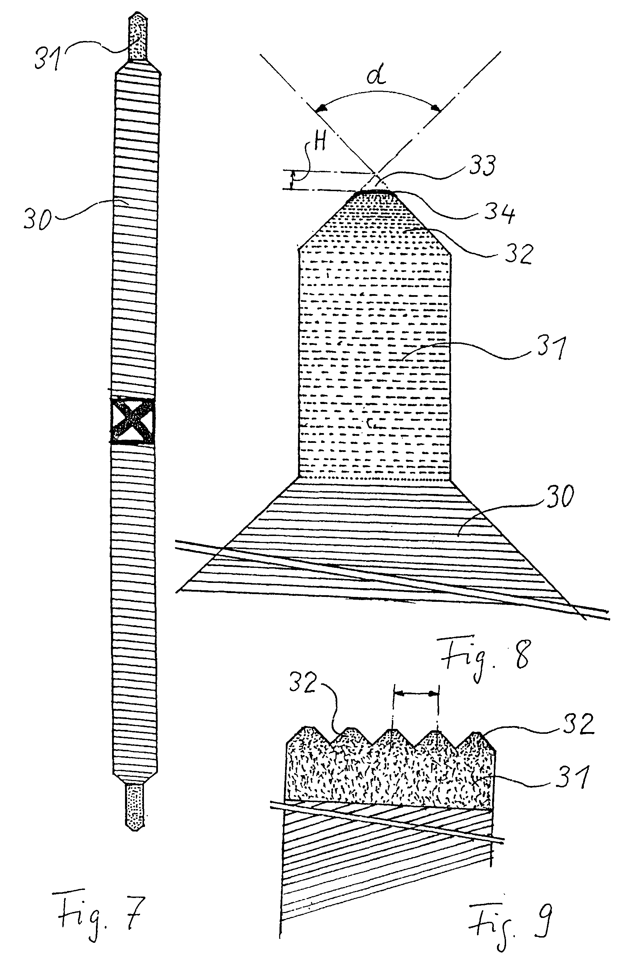

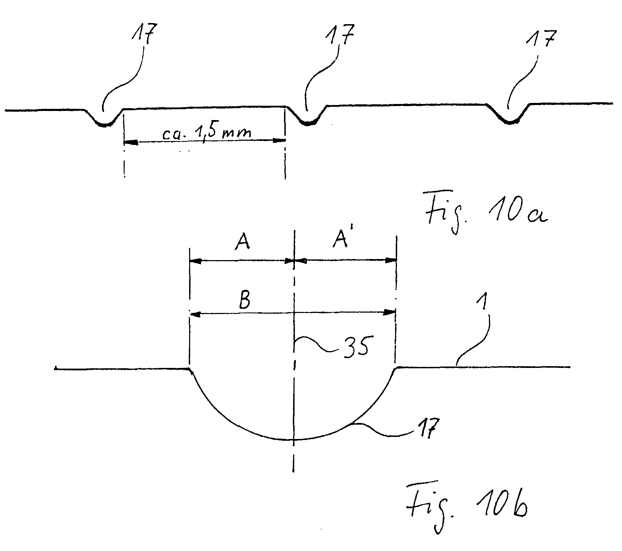

In Fig. 7 ist schematisch der Querschnitt einer runden Schleifscheibe 30 gezeigt, auf deren Peripherie ein gesinterter Diamant-Aufsatz 31 aufgebracht ist. Die Trägerscheibe 30 ist etwa 10 mm stark und hat einen Durchmesser von ca. 200 mm. Der Werkstoff der Trägerscheibe kann ein gehärteter armierter Epoxidharz sein, jedoch andere geeignete Materialien können hierfür ebenfalls verwendet werden. Auf der Peripherie der Trägerscheibe 30 befindet sich längs des Umfangs der gesinterte Diamantaufsatz 31 mit der erfindungsgemäßen Diamant-Schleifspitze 32. Der Diamantaufsatz 31 weist am Ende eine Spitze mit einem bestimmten Winkel (α) auf, die in Fig. 8 im vergrößerten Maßstab gezeigt ist. Der Winkel α ist im allgemeinen zwischen 60° und 120° zu wählen, je nachdem welche Art einer Ringnut benötigt wird. Der häufigste Anwendungsfall ist derjenige mit α = 90°. Um der Ringnut 17, 18 auf der Oberfläche des Tauchwerkzeugs 1 eine entsprechende Querschnittsform zu verleihen, ist der obere Teil 33 der Diamant-Schleifspitze 32 leicht abgestumpft, so daß die Kanten 34 der Schleifspitze rund sind. Die Höhe (H) der Abstumpfung 33 ist vorteilhaft zwischen 0,2 bis 0,6 mm, je nach Anwendungsfall zu wählen. Dabei ist es wichtig, daß an den Kanten ein gewisser Krümmungsradius, im vorliegenden Fall r = 0,4 mm, vorhanden ist.

In Fig. 9 ist ein weiteres Ausführungsbeispiel des Diamantaufsatzes 31 im Querschnitt gezeigt. Für den Fall, daß eine Vielzahl von Ringnuten 17, 18 nebeneinander geschliffen werden müssen, ist es zweckmäßig, mehrere Diamant-Schleifspitzen 32 auf einem Aufsatz 31 anzuordnen. Mit einem derartigen Aufsatz könnten somit fünf Ringnuten 17 bei einem Schleifvorgang hergestellt werden. Dies verkürzt um ein Vielfaches die Arbeitszeit zur Herstellung der geforderten Ringnuten 17, 18.

Die Körnung der Schleifspitzen 32 muß verhältnismäßig fein sein, damit in die Ringnut keine Riefen hineingezogen werden, in denen sich das elastische Material festsetzt. Typische Körnungen hierfür sind beispielsweise D10, D20 bis D60.

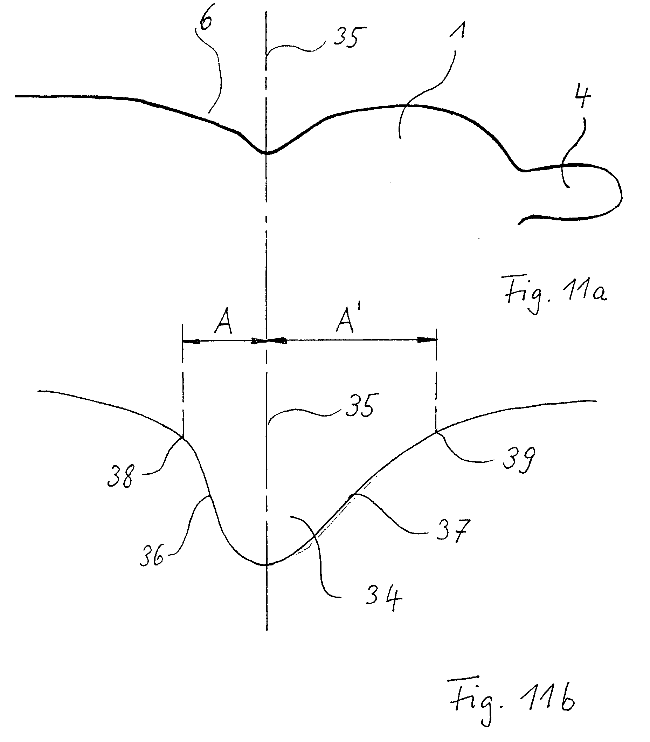

Die Fig. 10a zeigt einen Ausschnitt der Oberfläche eines Tauchwerkzeugs 1, in die symmetrische Ringnuten 17 mit den erfindungsgemäßen Diamant-Schleifspitzen 32 eingeschliffen wurden. Die typischen Breiten (B) der Öffnungen der symmetrischen Ringnuten 17 liegen zwischen 0,3 mm bis 0,6 mm (s. Fig. 10b). Die Abstände der einzelnen Ringnuten 17 untereinander sind frei wählbar, jedoch sollte der Abstand von 1,5 mm nicht wesentlich unterschritten werden.

In Fig. 11a ist der obere geformte Teil eines Tauchwerkzeugs 1 zur Hälfte dargestellt. In der Vertiefung 6 befindet sich am tiefsten Punkt eine erfindungsgemäße asymmetrische Ringnut 34, die mit der erfindungsgemäßen Diamant-Schleifspitze 32 eingeschliffen wurde. Die Asymmetrie dieser Ringnut 34 bezieht sich auf eine gedachte Mittelachse 35, die senkrecht auf der Längsachse des Tauchwerkzeugs 1 steht. Das charakteristische an dieser asymmetrischen Ringnut 34 sind die unterschiedlichen Steilheiten der die Ringnut begrenzenden Wände 36, 37 (s. Fig. llb). Dabei ist es wichtig, daß die in dieser Zeichnung links von der Mittelachse 35 liegende Wand 36 steiler ist als die auf der rechten Seite der Mittelachse 35 liegende Wand 37. Das Verhältnis der Abstände A, A' der Ringnutkanten 38, 39 zueinander sollte im Verhältnis A:A' = 1:2 stehen. Diese Asymmetrie im Querschnitt der Ringnut 34 ist bedeutend für das Fließverhalten des elastischen Materials (Latex) beim Austauchen aus dem Latex-Bad. Hierdurch wird verhindert, daß Lufteinschlüsse oder andere Unebenheiten in der Materialbeschaffenheit des herzustellenden Prophylaktikums entstehen.

Mit dem erfindungsgemäßen Schleifverfahren zur Herstellung von Tauchwerkzeugen, insbesondere der Ringnuten 17, 18, 34 auf der Oberfläche des Tauchwerkzeugs 1 ist es möglich, saubere und präzise Schliffe in der Oberfläche herzustellen, wodurch ein sauberes Verfließen des elastischen Materials gewährleistet ist, im Gegensatz zu anderen Herstellungsverfahren von Ringnuten, die beispielsweise auf chemischen Wege durch Ätzen hergestellt werden.

## Patentansprüche

1. Tauchwerkzeug (1) zur Herstellung eines Prophylaktikums mit einem länglichen zylindrischen Schaft (2) mit einem durch einen s-förmig geformten Abschnitt (3) und ein daran anschließenden Reservoirteil (4) verschlossenen Ende wobei der geformte Abschnitt (3) eine Vertiefung (6) aufweist, die aus zwei mit den kleinen Deckflächen aneinanderstoßenden Kegelstümpfen (8, 9) gebildet wird, und der Gradient in Längsrichtung der Oberfläche des Tauchwerkzeuges (1) stetig verläuft, **dadurch gekennzeichnet**, daß
- der Durchmesser (D5) des Tauchwerkzeugs (1) an der engsten Stelle der Vertiefung (6) zwischen 15 und 25 mm beträgt;
- der Krümmungsradius (R1) an der engsten Stelle (D5) der Vertiefung (6) zwischen 17 und 20 mm beträgt;
- der Öffnungswinkel des oberen, zum Reservoirteil (4) hin geöffneten Kegelstumpfes (9) 50° beträgt;
- der Öffnungswinkel des unteren Kegelstumpfes (8) 40° beträgt;
- der Krümmungsradius an der Übergangsstelle vom zylindrischen (2) zur Vertiefung (6) im geformten Abschnitt (3) konvex und größer 25 mm ist;
- die Gesamtlänge von der Vertiefung (6) bis zum Eingang in das Reservoirteil (4) zwischen 45 mm und 65 mm beträgt;
- der größte Durchmesser (D2) des geformten Abschnitts (3) im Bereich zwischen Vertiefung (6) und Eingangsbereich in das Reservoirteil (4) zwischen 33 und 35 mm beträgt.

2. Tauchwerkzeug (1) zur Herstellung eines Prophylaktikums mit einem länglichen zylindrischen Schaft (2) mit einem durch einen s-förmig geformten Abschnitt (3) und ein daran anschließenden, keulenartiges Reservoirteil (4) verschlossenen Ende, wobei der geformte Abschnitt (3) eine Vertiefung (6) aufweist, die aus zwei mit den kleinen Deckflächen aneinanderstoßenden Kegelstümpfen (8, 9) gebildet wird, und der Gradient in Längsrichtung der Oberfläche des Tauchwerkzeugs (1) stetig verläuft, **dadurch gekennzeichnet**, daß
- der konkave Krümmungsradius (R3) im Übergangsbereich (15) zwischen Reservoirteil (4) und geformtem Abschnitt (3) größer oder gleich 12 mm ist;
- die Tangente (15') im Wendepunkt der s-förmigen Kurve im Übergangsbereich (15) zwischen Reservoirteil (4) und geformtem Abschnitt (3) einen Winkel (β) zwischen 40° und 75° mit der Senkrechten einschließt.

3. Tauchwerkzeug nach Anspruch 2, **dadurch gekennzeichnet**, daß der Durchmesser (D3) im Eingangsbereich in das Reservoirteil (4) zwischen 6 mm und 11 mm im unteren Bereich und der maximale Durchmesser (D4) des Reservoirteils (4) zwischen 11 und 14 mm beträgt.

4. Tauchwerkzeug nach Anspruch 2, dadurch gekennzeichnet, daß der konvexe Krümmungsradius (R4) im Bereich des geschlossenen Endes des Reservoirteils (4) rund 6 mm beträgt.

5. Tauchwerkzeug nach Anspruch 2, **dadurch gekennzeichnet**, daß der Kurvenverlauf des keulenartigen Reservöirteils (4) eine gleichmäßige Verteilung des aufzubringenden elastischen Materials begünstigt.

6. Tauchwerkzeug nach einem der vorangegangenen Ansprüche, dadurch gekennzeichnet, daß im Bereich des engsten Durchmessers (D5) der Vertiefung (6) mindestens eine Ringnut (17, 18) angeordnet ist.

7. Tauchwerkzeug nach Anspruch 6, **dadurch gekennzeichnet**, daß bei mehr als einer Ringnut (17, 18) der gegenseitige Abstand der Ringnuten (17, 18) rund 1,5 mm beträgt.

8. Tauchwerkzeug nach einem der vorangegangenen Ansprüche, **dadurch gekennzeichnet**, daß die Ringnuten (17, 18) symmetrisch zur Mittelachse (35) geformt werden.

9. Tauchwerkzeug nach einem der vorangegangenen Ansprüche, dadurch gekennzeichnet, daß die Ringnuten (34) asymmetrisch zur Mittelachse (35) derart geformt werden, daß die Wand (36) der Ringnut (34) links der Achse (35) steiler als die Wand (37) rechts der Achse (35) verläuft.

10. Tauchwerkzeug nach einem der vorangegangenen Ansprüche, dadurch gekennzeichnet, daß die Abstände (A, A') zur Mittelachse (35) der Ringnut (34) etwa das Verhältnis A:A' gleich 1:2 beträgt.

11. Verfahren zur Herstellung eines Prophylaktikums, wobei das Prophylaktikum aus einem länglichen zylindrischen Schaft (2) mit einem durch einen s-förmig geformten Abschnitt (3) und ein daran anschließenden Reservoirteil (4) verschlossenen Ende und aus einem Formteil aus dünnwandigem elastischem Material besteht, das in Tauchbädern auf ein an die Form des Prophylaktikums angepaßten Tauchwerkzeugs äußerlich aufgebracht wird und das fertige Prophylaktikum mittels gegensinnig rotierender Bürsten (25, 26) und mindestens eins auf das Tauchwerkzeug (1) einwirkenden Wasserstrahls (27, 28) abgestreift wird, **dadurch gekennzeichnet**, daß
- das Tauchwerkzeug (1) in zwei Tauchvorgängen (10, 13) um seine Längsachse rotierend in einer Schräglage zur Oberfläche des elastischen Materials in das aufzubringende elastische Material eingetaucht wird; und
- nach den Tauchvorgängen (10, 13) jeweils ein Trocknungsvorgang (23, 24) vorgesehen ist.

12. Verfahren nach Anspruch 8, **dadurch gekennzeichnet**, daß der mindestens eine Wasserstrahl (27, 28) so ausgerichtet ist, daß er hinter dem Rollring (7) während des Abstreifvorgangs direkt auf das Tauchwerkzeug (1) einwirkt.

## Claims

1. Plunger tool (1) for producing a prophylactic with an oblong cylindrical shaft (2) with a section (3) formed S-shaped, and a reservoir section (4) adjoining said section (3) and having a closed end, whereby the shaped section (3) has a narrowing (6) f ormed by two blunt cones (8, 9) abutting each other with their covering surfaces, and the gradient extends constant in the longitudinal dorection of the surface of the plunger tool (1), characterized in that
- the diameter (D5) of the plunger tool (1) at the narrowest point of the narrowing (6) amounts to between 15 and 25 mm;
- the radius of curvature (R1) at the narrowest point (D5) of the narrowing (6) amounts to between 17 and 20;
- the opening angle of the upper blunt cone (9), the latter being open toward the reservoir section (4), amounts to 50°;
- the opening angle of the lower blunt cone (8) amounts to 40°;
- the radius of curvature at the point of transition from the cylindrical section (2) to the narrowing (6) in the shaped section (3) is convex and greater than 25 mm;
- the total length from the narrowing (6) to the inlet into reservoir section (4) is between 45 mm and 65 mm; and
- the largest diameter (D2) of the shaped section (3) within the zone between the narrowing (6) and the inlet zone of reservoir section (4) amounts to between 33 and 35 mm.

2. Plunger tool (1) for producing a prophylactic with an oblong cylindrical shaft (2) with a section (3) formed S-shaped and a club-like reservoir section (4) adjoining the latter and having a closed end, whereby the shaped section (3) has a narrowing (6) formed by two blunt cones (8, 9) abutting each other with their covering surfaces, and the gradient extends constant in the longitudinal direction of the surface of the plunger tool (1), characterized in that
- the concave radius of curvature (R3) in the zone of transition between the reservoir section (4) and the shaped section (3) is greater than or equal to 12 mm;
- the tangent (15') at the point of reversal of the s-shaped curve in the zone of transition (15) between the reservoir (4) and shaped section (3) encloses an angle ( ) between 40 and 75 relative to the longitudinal axis.

3. Plunger tool according to claim 2, characterized in that the diameter (D3) in the inlet zone of reservoir section (4) is between 6 mm and 11 mm in the lower zone, and the maximum diameter (D4) of the reservoir section (4) amounts to between 11 and 14 mm.

4. Plunger tool according to claim 2, characterized in that the convex radius of curvature (R4) within the zone of the closed end of reservoir section (4) comes to about 6 mm.

5. Plunger tool according to claim 2, characterized in that the course of the curve of the club-like reservoir section (4) promotes uniform distribution of the elastic material to be applied.

6. Plunger tool according to any one of the preceding claims, characterized in that at least one annular groove (17, 18) is arranged within the zone of the narrowest diameter (D5) of the narrowing (6).

7. Plunger tool according to claim 6, characterized in that with more than one annular groove (17, 18), the spacing between the annu]ar grooves (17, 18) amounts to about 1.5 mm.

8. Plunger tool (1)according to any one of the proceding claims, characterized in that the annular grooves (17, 18) are shaped symmetrically to the longitudinal axis (35) of the plunger tool (1).

9. Plunger tool (1)according to any one of the proceding claims, characterized in that the annular grooves (34) are shaped asymmetrically to the longitudinal axis (35) of the plunger tool (1) so that the wall (37) of the annular grooves (34) left to the longitudinal axis (35) are steeper than the wall (37) right to the longitudinal axis (35).

10. Plunger tool (1)according to any one of the proceding claims, characterized in that the distances (A, A') between the longitudinal axis (35) of the annular groove (34) are about A : A' as 1 : 2 .

11. Process for producing a prophylactic, whereby the prophylactic consists of an oblong cylindrical shaft (2) with a section (3) formed S-shaped, and a reservoir section (4) adjoining the latter and having a closed end, and a shaped section made of thin-walled elastic material, which is externally applied to a plunger tool adapted to the shape of the prophylactic, and the finished prophylactic is stripped off by means of brushes (25,26) rotating in opposite direction and at least one water jet (27, 28) acting on the plunger tool (1) during the stripping operation, charactereized in that
- the plunger tool (1) is immersed in two operations (10, 13)in the elastic material to be applied , rotating around ist longitudinal axis in a position inclined relative to the surface of the elastic material; and
- after the immersion opraration (10,13) a first and second drying step (23, 24) takes place.

12. Process according to claim 8, characterized in that the at least one water jet (27, 28) is directed in such a way that it directly acts on the plunger tool (1) behind the rolling ring (7) during the stripping operation.

## Revendications

1. Outil a immergé (1) pour la fabrication d'un prophelactic avec une queue cylindrique en longueur (2) avec une section moulée en s (3) et, dans le prolongement, une partie renflée réservoir (4) sur la partie fermée, la partie moulée (3) présentant un évidement (6) formé par deux bouts sphériques (8, 9) venant s'effleurer dans la partie de recouvrement de plus petite surface, l'inclinaison de la surface de l'outil immergé (1) dans le sens de la longueur étant constante, caractérisé en ce que
- le diamètre (D5) de l'outil immergé (1) est de 15 à 25 mm dans la partie la plus étroite de l'évidement (6) ;
- le rayon de courbure (R1) de la partie la plus étroite (D5) de l'évidement (6) est de 17 à 20 mm ;
- l'angle d'ouverture du bout sphérique (9) supérieur, ouvert vers la partie réservoir (4), est de 50° ;
- l'angle d'ouverture du bout sphérique inférieur (8) est de 40° ;
- l'angle d'ouverture de la transition entre la partie cylindrique (2) et l'évidement (6) dans la partie moulée (3) est convexe et supérieure à 25 mm ;
- la longueur totale de l'évidement (6) jusqu'à l'entrée dans la partie réservoir (4) est de 45 mm à 65 mm ;
- le plus grand diamètre (D2) de la section moulée (3) dans la zone entre l'évidement (6) et la zone d'entrée dans la partie réservoir (4) est de 33 à 35 mm.

2. Outil a immergé (1) pour la fabrication d'un protecteur avec une queue cylindrique en longueur (2) avec une section moulée en s (3) et, dans le prolongement, une partie renflée réservoir (4) sur la partie fermée, la partie moulée (3) présentant un évidement (6) formé par deux bouts sphériques (8, 9) venant s'effleurer dans la partie de recouvrement de plus petite surface, l'inclinaison de la surface de l'outil immergé (1) dans le sens de la longueur étant constante, caractérisé en ce que
- le rayon de courbure (R3) concave dans la zone de transition (15) entre la partie réservoir (4) et la partie moulée (3) est supérieur ou égal à 12 mm ;
- la tangente (15') au point d'inversion de la courbe en s dans la zone de transition (15) entre la partie réservoir (4) et la partie moulée (3) forme un angle β entre 40° et 75° par rapport à la verticale.

3. Outil a immergé selon la revendication 2, caractérisé en ce que le diamètre (D3) dans la zone d'entrée de la partie réservoir (4) est de 6 mm à 11 mm dans la partie inférieure et que le diamètre maximal (D4) de la partie réservoir (4) est de 11 à 14 mm.

4. Outil immergé selon la revendication 2, caractérisé en ce que le rayon de courbure (R4) de la zone de l'extrémité fermée de la partie réservoir (4) est de 6 mm approximativement.

5. Outil immergé selon la revendication 2, caractérisé en ce que le profil courbe de la partie réservoir renflée (4) facilite la distribution régulière du matériau élastique devant être appliqué.

6. Outil immergé selon l'une des revendications précédentes, caractérisé en ce qu'au moins une rainure circulaire (17, 18) est aménagée dans la partie de plus faible diamètre (D5) de l'évidement (6).

7. Outil immergé selon la revendication 6, caractérisé en ce que dans le cas de plus d'une rainure circulaire (17, 18) l'écart entre les rainures circulaires (17, 18) est d'approximativement 1,5 mm.

8. Outil immergé selon l'une des revendications précédentes, caractérisé en ce que les rainures circulaires (17, 18) ont une géométrie symétrique par rapport à l'axe médian (35).

9. Outil immergé selon l'une des revendications précédentes, caractérisé en ce que les rainures circulaires (34) ont une géométrie asymétrique par rapport à l'axe médian (35), de manière que la paroi (36) de la rainure circulaire (34) présente une plus forte inclinaison à gauche de l'axe (35) que la paroi (37) à droite de l'axe (35).

10. Outil immergé selon l'une des revendications ci-dessus, caractérisé en ce que les écarts (A, A') de la rainure circulaire (34) par rapport à l'axe médian (35) sont dans une proportion A:A' de 1:2 approximativement.

11. Procédé pour la fabrication d'un protecteur, le protecteur étant constitué d'une queue cylindrique (2) avec une section moulée en forme de s (3) et une partie
réservoir (4) dans la section d'extrémité en prolongement et une pièce moulée à paroi mince en matériau souple qui est adaptée extérieurement dans les bains d'immersion à un outil a immergé adapté à la forme du protecteur et qui racle le protecteur assemblé au moyen de brosses animées d'un mouvement rotatif de sens contraire (25, 26) et au moins un jet d'eau (27, 28) dirigé contre l'outil a immergé (1), caractérisé en ce que
- l'outil immergé (1) est plongé dans le matériau souple devant être adapté en deux phases d'immersion (10, 11) par rotation dans le sens axial en position oblique par rapport à la surface du matériau souple ; et
- une phase de séchage (23, 24) est prévue après chaque phase d'immersion (10, 13).

12. Procédé selon la revendication 8, caractérisé en ce qu'au moins un jet d'eau (27, 28) est orienté de telle manière qu'il soit appliqué directement contre l'outil immergé (1) derrière la bague rotative (7) pendant le raclage.
